(19) Europäisches Patentamt
European Patent Office
Office européen des brevets

(11) **EP 2 246 433 A1**

(12) **EUROPÄISCHE PATENTANMELDUNG**

(43) Veröffentlichungstag:
**03.11.2010 Patentblatt 2010/44**

(51) Int Cl.:
*C12N 15/117* (2010.01)  *A61K 31/713* (2006.01)

(21) Anmeldenummer: **09075220.5**

(22) Anmeldetag: **30.04.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**AL BA RS**

(71) Anmelder: **Mologen AG**
**14195 Berlin (DE)**

(72) Erfinder:
• **Kleuss, Christiane**
 **12203 Berlin (DE)**
• **Schroff, Matthias**
 **14195 Berlin (DE)**

• **Schmidt, Manuel**
 **14195 Berlin (DE)**
• **Wittig, Burghardt**
 **14129 Berlin (DE)**

(74) Vertreter: **Harrison, Robert John et al**
 **24IP Law Group Sonnenberg Fortmann**
 **Herzogspitalstraße 10a**
 **80331 München (DE)**

Bemerkungen:
Die Patentansprüche 18-19 und 21-24 gelten als fallen gelassen, da die entsprechenden Anspruchsgebühren nicht entrichtet wurden (R. 45 (3) EPÜ).

(54) **Concatemere zur Immunmodulation**

(57)   Die Erfindung betrifft ein polymeres, nicht-codierendes Nukleinsäuremolekül zur Modulation der Aktivität des menschlichen oder tierischen Immunsystems sowie ein Herstellungsverfahren desselben und ein Vakzin, die das polymere, nicht-codierende Nukleinsäuremolekül enthält, wobei polymere, nicht-codierende Nukleinsäuremoleküle als nicht-codierende Nukleinsäuremoleküle verstanden werden können, die aus vier nicht-codierenden monomeren Bausteinen bestehen (Tetramer) oder aber Assemblate aus noch mehr nicht-codierenden Nukleinsäuremolekülen sind (höhermolekulare Polymere), die kovalent miteinander verbunden sind.

Abb. 1

für i: 0..n    $n \in IN_0$    ·········· kovalente Bindung

**EP 2 246 433 A1**

**Beschreibung**

Die Erfindung betrifft ein polymeres, nicht-codierendes Nukleinsäuremolekül zur Modulation der Aktivität des menschlichen oder tierischen Immunsystems sowie ein Herstellungsverfahren desselben und ein Vakzin, die das polymere, nicht-codierende Nukleinsäuremolekül enthalten, wobei polymere, nicht-codierende Nukleinsäuremoleküle als nicht-codierende Nukleinsäuremoleküle verstanden werden können, die aus mindestens vier miteinander kovalent verbundener Moleküle (Tetramer) dieser nicht-codierenden Nukleinsäuremolekülen bestehen.

Während die adaptive Immunantwort nach Selektion der für das jeweilige Pathogen spezifischen Lymphozyten und deren klonaler Expansion und Differenzierung zu Effektorzellen erst verzögert (3-5 Tage) einsetzt, dann aber lang anhaltenden Schutz vor dem jeweiligen Pathogen durch Ausbildung eines "immunologischen Gedächtnisses" bietet, erkennen die Zellen des angeborenen Immunsystems Pathogene anhand konservierter Pathogen-assoziierter molekularer Muster (pathogenassociated molecular patterns = PAMPs) mit Keimzell-codierten Rezeptoren und reagieren sofort. Zu den für verschiedene Zelltypen unterschiedlichen Reaktionen gehören die Sekretion von Cytokinen (z.B. IL-1, IL-6, TNF-$\alpha$) und Chemokinen (z.B. IL-8/CXCL8, MIP-1$\alpha/\beta$, MCP-1), Aktivierung von Effektormechanismen (Phagocytose, respiratorische Entladung, Freisetzung bakterizider oder cytotoxischer Substanzen aus lytischen Granula), Expression von co-stimulatorischen Molekülen (CD80, CD86) sowie verstärkter Expression von MHC-Molekülen. Dadurch werden zum Einen Effektorzellen rekrutiert und aktiviert, die das eingedrungene Pathogen eliminieren können, zum Anderen erhalten die Zellen des adaptiven Immunsystems die für ihre Aktivierung notwendigen Signale.

Zur Erzeugung einer verbesserten Immunantwort wurden CpG-Oligonukleotide (CpG-ODN) als neue Klasse von immunmodulatorischen Molekülen eingesetzt. Solche nicht methylierten CG-Motive kommen in bakterieller DNA vor und stellen für das Immunsystem ein "danger signal" dar. Als "pathogen associated molecular pattern" (PAMP) bewirken sie vor allem die unspezifische Aktivierung des angeborenen Immunsystems (Krieg, Nat. Med 2003, 9: 831-835).

CpG-ODN induzieren über die Cytokine Interleukin-12, Interferon-$\gamma$ und Tumornekrosefaktor-$\alpha$ eine $T_H$1-betonte Immunantwort.

Immunstimulatorische Nukleinsäuren (ISS), die die benannten CpG-ODN tragen, sind nur einige Basen lang und enthalten keine offenen Leserahmen für die Expression von Proteinen.

Bei den ISS handelt es sich um lineare Nukleinsäuremoleküle, deren Enden offen sind (freie Hydroxyl- oder Phosphatgruppe) oder durch synthetische Gruppen geschützt sind.

Die Stimulation der zellulären Immunantwort ermöglicht eine Einflussnahme auf Regelkreisläufe, die ohne Eingriff nicht zu einer für den Patienten befriedigenden Immunaktivität führen.

Die Modifikation von CpG-ODN mit Phosphorothioat-Rückgrat, wie sie zur Stabilisierung von "CpG-DNA" eingesetzt wird, weist einige gravierende Nachteile auf. Hierzu gehören insbesondere die beobachtete Toxizität [Heikenwalder 2004, Levin 1999] sowie unspezifische Bindung an Proteine [Brown 1994].

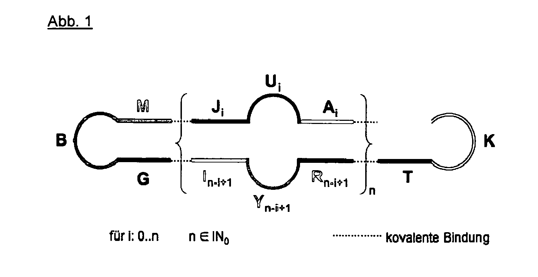

Aus diesem Grund wurde eine neue Klasse kovalent geschlossener immunmodulatorischer DNA-Moleküle entwickelt (WO 01/07055 / EP 1196178). Diese DNA-Moleküle bestehen aus zwei chemisch synthetisierten DNA-ODN, die einen selbstkomplementären Bereich am 5'- und am 3'-Ende mit palindromischen, überlappenden Enden aufweisen, so dass durch Ligation der beiden DNA-Moleküle ein kovalent geschlossenes Molekül entsteht. Diese DNA-Moleküle mit CG-Motiven im nichtkomplementären Bereich zeigen ähnliche Aktivität wie CpG-ODN (verstärkte Expression der Oberflächenmoleküle CD80, CD40, MHC auf B-Zellen und Sekretion von IL-6, IFN-$\gamma$, IFN-$\alpha$, IL-12, TNF$\alpha$ durch PBMC), weisen aber im Vergleich zu CpG-ODN mit Phosphorothioat-Rückgrat Unterschiede im Expressionsmuster der induzierten Cytokine und eine deutlich geringere Toxizität in Mäusen auf. Diese immunmodulatorische DNA des Standes der Technik weist bezüglich der Modulation der Aktivität des menschlichen oder tierischen Immunsystems aber einige Nachteile auf. Es ist nicht immer möglich, die Aktivität des menschlichen oder tierischen Immunsystems in der gewünschten Stärke zu modulieren, insbesondere zu aktivieren. Die Moleküle gemäß der WO 01/07055, wie sie beispielsweise in Figur 1 oder in Anspruch 11 dargelegt sind, bestehen aus mehreren Desoxyribonukleotidresten, die ein teilweise einzelsträngiges hantelförmiges und kovalent geschlossenes DNA-Molekül bilden, das im Sinne der hier vorgelegten Erfindung als Dimer bezeichnet wird. Gemäß der WO 01/07055 wurden die als Ausgangssubstanz verwendeten monomeren Nukleinsäuren vor der Ligation erhitzt, was dazu führte, dass einheitliche Moleküle aus Nukleinsäuren gebildet werden, die jeweils aus einem hantelförmigen Dimer (siehe auch Fig. 1 der WO 01/07055) bestehen. Das gebildete Nukleinsäuremolekül ist eine Hantel gemäß Fig. 1 der WO 01/07055. Monomer im Sinne der Erfindung bedeutet daher nicht eine Struktur aus z. B. einer einzigen Base, sondern bezeichnet eine Nukleinsäure, die selbst aus mehreren Desoxyribonukleotidmonophosphaten besteht (siehe Fig. 1 oder Anspruch 11 der WO 01/07055), die durch ihre definierte Basenabfolge oder eine definierte dreidimensionale Konformation gemeinsam erst die Monomer-typischen Eigenschaften ausbilden.

Ausgehend von diesem Stand der Technik ist es die Aufgabe der vorliegenden Erfindung, geeignete immunmodulatorische DNA-Moleküle, die zur Auslösung einer verbesserten Immunantwort fähig sind, sowie ein Verfahren zu ihrer Herstellung sowie Impfstoffe bereitzustellen, die diese immunmodulatorischen DNA-Moleküle enthalten.

EP 2 246 433 A1

Immunmodulation heißt im Zusammenhang mit dieser Erfindung, dass die Mittler- und Effektorzellen des Immunsystems, also vor allem die zur Zeit bekannten Thymozyten mit Helferfunktion und die cytotoxischen Thymozyten, B-Zellen und sog. NK (natural killer)-Zellen, Makrophagen und Monozyten sowie dendritische Zellen und ihre Vorläufer, sowie bisher in ihrer Funktion nicht aufgeklärte Zellpopulationen mit Funktionen innerhalb des Immunsystems, durch die Verwendung von Nukleinsäuremolekülen zur Proliferation, Migration, Differenzierung oder Aktivität angeregt werden. Immunmodulation heißt, dass neben einer generellen Verbesserung der Immunantwort im oben definierten Sinne auch die Art oder der Charakter einer Immunreaktion beeinflusst wird, sei es, dass eine im Entstehen oder der Reifung begriffene Immunreaktion davon betroffen ist, sei es, dass eine schon etablierte Reaktion in ihrem Charakter verändert wird.

Das in der vorliegenden Erfindung beanspruchte Molekül mit verbesserter immunmodulierenden Eigenschaften gegenüber den dimeren Substanzen aus WO 01/07055 ist ein polymeres, nicht-codierendes Nukleinsäuremolekül. Unter einem polymeren Nukleinsäuremolekül soll ein sogenanntes höhermolekulares Concatemer verstanden werden. Das erfindungsgemäße polymere Molekül ist herstellbar durch ein Verfahren, welches die folgenden Schritte umfasst:

-    Bereitstellung einer 5'-phosphorylierten Desoxyribonukleinsäure,

-    Alkoholfällung und anschließende Trocknung des Präzipitats bei 50°C, oder Lyophylisierung des DNA-Molelüls bei 50°C, insbesondere in Gegenwart von MgCl$_2$, bis ein trockener Rückstand erhalten wird, und anschließend Resuspension in einer Pufferlösung,

-    Zugabe einer T4-DNA Ligase, wodurch ein Reaktionsgemisch gebildet wird und

-    Inkubation des Reaktionsgemisches bei 37°C für mindestens 30 Minuten.

Concatemere bestehen aus kovalent miteinander verbundenen Monomer-Einheiten, die in ihrer Gesamtheit circulär geschlossen sind, im Abstand der aufbauenden Monomere doppelsträngige Bereiche und immunmodulatorische CG-Motive bevorzugt in den einzelsträngigen Bereichen aufweisen. Es war völlig überraschend, dass diese Polymere, bestehend aus Tetrameren, Hexameren oder höhermolekularen Assemblaten von kovalent geschlossener immunmodulatorischer DNA einen überraschend verbesserten Effekt gegenüber den dimeren Molekülen des Standes der Technik haben.

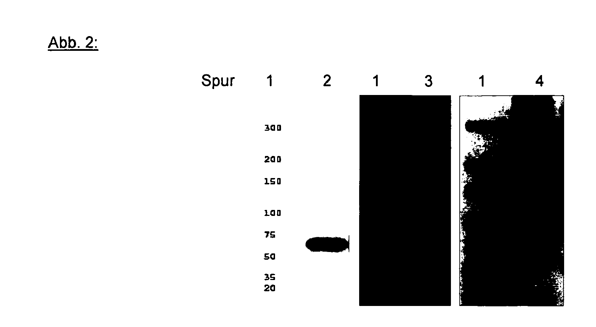

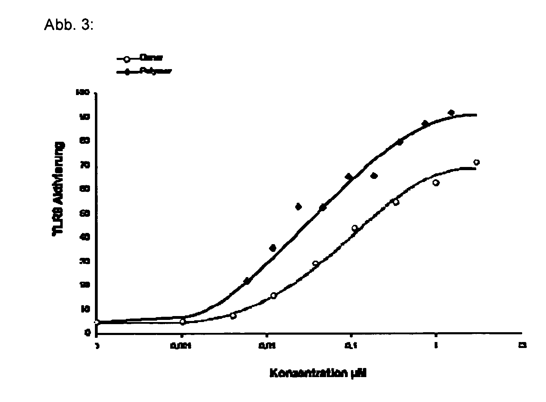

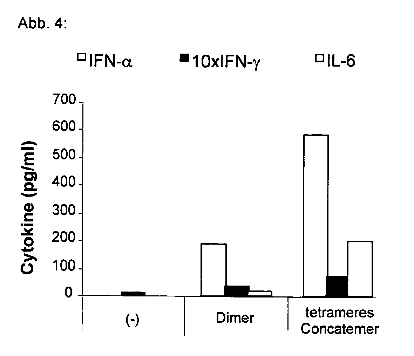

Die beanspruchten polymeren Moleküle werden durch die in Abb. 1 und 2 dargestellten molekularen und die in Abb. 3 und 4 gezeigten funktionellen Eigenschaften definiert, die sich aus der Anwendung des Verfahrens seiner Herstellung für den Fachmann ergeben. Bei dem beschriebenen Verfahren werden bei Einsatz von Nukleinsäuremolekülen mit palindromischen 5'- bzw. 3'-Enden als Edukte Polymere verschiedener Größe dargestellt, von denen nur die hier beanspruchten Tetramere und höhermolekularen Assemblate die hochpotenten Funktionen erfüllen. Da die Kennzeichnung durch strukturelle Merkmale aufgrund der ausgedehnten und verschiedenartigen Molekülgröße nicht praktikabel ist, ist die Darstellung der Polymere über den Herstellungsprozess besonders präzise. Durch das neue Verfahren wird ein anderes Erzeugnis als das im Stand der Technik beschriebene bereitgestellt. Dies kann durch deutliche Unterschiede in den Eigenschaften der Dimere und den erfindungsgemäßen Polymeren gemäß Abb. 3 gezeigt werden. Die erfindungsgemäßen höhermolekularen Polymere sind überraschend besser zur Immunmodulation geeignet als die nichtpolymeren Strukturen des Standes der Technik.

Die erfindungsgemäßen Moleküle können auch durch die Bereitstellung von 5'-phosphorylierten Desoxyribonukleinsäuren in Wasser hergestellt werden, wenn diese mit einem zu einer Polyacrylamidgelelektrophorese äquivalenten Verfahren aufgereinigt werden, insbesondere durch eine kombinierte Reinigung mit einer HPLC gefolgt von einer FPLC. Dem Fachmann ist bekannt, dass durch die Kombination von mehreren Hochleistungsreinigungsverfahren wie HPLC oder FPLC ein zu einem PAGE-Reinigungsgrad analoger Reinigungsgrad erzielt werden kann. Überraschenderweise führt die Abfolge der einzelnen Verfahrensschritte zu einem polymeren Molekül, das sich circulär stabil aus seinen kovalent miteinander verbundenen Monomeren zusammensetzt und aus wenigstens 24 Nukleotiden besteht. Die gleichzeitig entstehenden höhermolekularen Polymere enthalten immer eine geradzahlige Anzahl der monomeren Bausteine. Die gebildete Molekülkette enthält keine freien 5' oder 3' Enden. Die das erfindungsgemäße Molekül durch intermolekulare Veresterung bildenden Monomere sind wie folgt charakterisiert:

-    sie enthalten einen Bereich von wenigstens 2 sequentiellen Nukleotiden, der unter geeigneten Bedingungen mit einem anderen Bereich dieses Monomers eine doppelsträngige Stammstruktur bilden,

-    zwischen diesen beiden revers komplementären Bereichen liegen wenigstens 4 Nukleotide,

-    CG-Motive, die von zellulären Strukturen erkannt werden, befinden sich bevorzugt im einzelsträngigen Bereich,

- ebenso können sich im einzelsträngigen Bereich modifizierte Nukleotide befinden, die kovalent mit Fettsäuren, Zuckern oder Aminosäuren verbunden sind.

Ein erfindungsgemäßes Molekül besteht aus wenigstens vier Monomeren und wird konformativ während der oben genannten Synthese gebildet. Die Monomere fügen sich zu Ketten von zwei, vier, sechs und mehr durch Ausbildung kovalenter Bindungen zusammen. Es werden dann sogenannte Di-, Tetra- oder Hexamere gebildet, von denen alle außer den Dimeren als Polymere bezeichnet werden sollen.

Das erfindungsgemäße Molekül kann als Concatemer definiert werden. In einer besonders bevorzugten Ausführungsform ist vorgesehen, dass das erfindungsgemäße Molekül ein concatemeres Molekül ist, bei dem mindestens vier Loop-Bestandteile individueller Monomere miteinander verbunden, bevorzugt linear aneinander gefügt verbunden sind, so dass bevorzugt zwei, besonders bevorzugt mehrere doppelsträngige Bereiche jeweils durch einzelsträngige Loop-Elemente voneinander separiert vorliegen.

Die erfindungsgemäßen Moleküle sind in der Lage, die Aktivität des menschlichen oder tierischen Immunsystems besser zu modulieren als Moleküle des Standes der Technik. Die Moleküle des Standes der Technik sind die bekannten immunmodulatorischen Nukleinsäuren, die als niedermolekulare hantelförmige Strukturen wirken. Die meisten bekannten, immunmodifizierenden kurzen Oligodesoxyribonukleinsäuren enthalten ein unmethyliertes Cytosin-Guanosin-Motiv. Die physiologische Wirkung dieser Nukleinsäuren wird als Immunmodulation bzw. Modulation der Aktivität des Immunsystems im Sinne der Erfindung verstanden. In der EP 1 196 178 werden darüber hinaus verschiedene Moleküle offenbart, die aus einem Stamm und mindestens einer Schleife bestehen, wie sie beispielsweise in den Figuren 1 und 2 der EP 1 196 178 offenbart sind. Im Sinne der vorliegenden Erfindung handelt es sich bei diesen Molekülen um eine Dimerstruktur. Die vorliegende Erfindung umfasst derartige Dimere nicht. Hierbei ist zu berücksichtigen, dass der Begriff des Polymers in der Wissenschaft mehrfach mit unterschiedlichen Bedeutungen verwendet wird. Ein Polymer kann beispielsweise eine längere Nukleinsäure sein, aber auch eine Struktur, die aus mehreren gleichen oder ähnlichen Molekülen besteht und zu einem größeren Assemblat zusammengefügt wurde. Unter Polymer sollen im Sinne der Erfindung Molekülketten verstanden werden, die aus mindestens 4 Monomeren bestehen. Bei Verwendung der bevorzugten Monomere entspricht das Molekulargewicht des resultierenden Tetramers ungefähr 170 kDa (siehe Abb. 2). Polymere im Sinne der Erfindung wären beispielsweise mehrere Stamm-Schleifen-Strukturen wie in Abb. 1 dargestellt, die mit mehreren gleichen oder ähnlichen Stamm-Schleifen-Strukturen zu einer höheren Struktur (einem Polymer) zusammengefügt wurden. Polymer werden alle erfindungsgemäßen Moleküle bezeichnet, die größer als 23 kDa sind. Durch die aufgezeigten Reaktionsbedingungen erfolgt während der Ligation eine transiente Aneinanderlagerung der Monomere, die dann durch die Ligase verestert werden. Ein so entstandenes Polymer wird konformativ während der Synthese nur unter speziellen Reaktionsbedingungen gebildet. Es ist nicht möglich, höhermolekulare Polymere aus einmal gebildeten Dimeren herzustellen. Die das Polymer bildenden Monomerstrukturen sind kovalent miteinander verbunden. Das einmal entstandene Polymer ist stabil gegen Hitze und denaturierende Agenzien, was auch umgekehrt bedeutet, dass die Dimeren nicht mehr mit einfachen physikalischen Mitteln aus den erfindungsgemäßen höhermolekularen Molekülen gewonnen werden können.

Es ist überraschend, dass derartige polymere Strukturen, die verbesserte und nicht naheliegende Eigenschaften gegenüber den Dimerstrukturen haben, durch vergleichsweise einfache Verfahrensschritte gewonnen werden können. Die Gewinnung der höhermolekularen Assemblate kann beispielsweise durch Zentrifugation, Gelelektrophorese oder Säulenchromatographie erfolgen, mithilfe derer hochkomplexe Strukturen, wie beispielsweise Tetramere, Hexamere oder andere, nachgewiesen und gewonnen werden können, die gegenüber den Dimeren überraschend bessere Eigenschaften bei der Modulation des Immunsystems zeigen (siehe Abb. 3 und 4). Dies zeigt sich darin, dass sie im Labororganismus oder beim Menschen zu einer anderen Form der Immunmodulation führen.

Alle Desoxyribonukleinsäuren, die der folgenden Charakterisierung entsprechen, können im Polymerisierungsverfahren eingesetzt werden.

5'-P-W-S-3', wobei

· P, W, S Nukleinsäuren sind, die miteinander in der gelisteten Lesereihenfolge über Phosphodiesterbindungen "-" verbunden sind,
· die Sequenz der Nukleinsäure P, W oder S mindestens ein Motiv der Desoxyribonukleotidabfolge CG enthält,
· W mindestens 4 Nukleotide lang ist und
· Sequenzen der Nukleinsäureabschnitte S und P revers komplementär zueinander sind.

Die resultierenden Polymere genügen dann der folgenden Formel:

$$W\text{-}S\text{-}\{P\text{-}W\text{-}S\}_n\text{-}P\text{-}W\text{-}S\text{-}\{P\text{-}W\text{-}S\}_n\text{-}P$$

$\forall\, n \in IN_0$, wobei

· die in der Formel geschriebene rechte Nukleinsäure P kovalent mit der linken Nukleinsäure W verbunden ist und

· "n" den Grad der Concatemerisierung durch Angabe der Anzahl der inneren Monomer-Einheiten angibt.

Da die hier beanspruchten Polymere ihre Eigenschaften durch ihre Konformation erhalten, können polymere Strukturen im Sinne dieser Anmeldung auch aus nicht sequenzidentischen Monomeren aufgebaut sein. Hierbei kann die Nukleinsäuregruppe W Moleküle mit den Sequenzen von B, U, K, Y, die Nukleinsäuregruppe P Moleküle mit den Sequenzen von J, E, R, G und die Nukleinsäuregruppe S Moleküle mit den Sequenzen von M, A, T, I enthalten. In Abhängigkeit von der Vielzahl n der Monomere im Kernbereich des gebildeten Polymer gibt es mehrere verschiedene Sequenzabschnitte J-U-A bzw. R-Y-I, die auch untereinander nicht sequenzidentisch sein müssen, was durch den Index "i" bzw. "n-i+1" angezeigt wird. Die beanspruchten Polymere mit sequenzidentischen oder sequenzverschiedenen Monomerbausteinen genügen dann der allgemeinen Formel:

$$B\text{-}M\text{-}\{[J_i\text{-}U_i\text{-}A_i]_{0..n}\}_n\text{-}E\text{-}K\text{-}T\text{-}\{[R_{n-i+1}\text{-}Y_{n-i+1}\text{-}I_{n-i+1}]_{0..n}\}_n\text{-}G$$

$\forall\, n \in IN_0$ , wobei

· A, B, E, G, I, J, K, M, R, T, U, Y Desoxyribonukleotid-Moleküle sind und
· die Sequenz des i-ten Bausteins eines Nukleinsäuremoleküls von der (i+1)-ten des gleichen Moleküls verschieden sein kann aber nicht muß und
· mindestens eine der Nukleinsäuren ein Motiv mit der Desoxyribonukleotidabfolge CG enthält und
· B, $U_i$, K und $Y_{n-i+1}$ überwiegend einzelsträngig vorliegen und
· B, $U_i$, K und $Y_{n-i+1}$ aus jeweils mindestens 4 Desoxyribonukleotiden aufgebaut sind und
· sich die Sequenzen von $J_i$ zu $I_{n-i+1}$, $A_i$ zu $R_{n-i+1}$, M zu G bzw. E zu T revers kompementär verhalten und
· G mit B kovalent über eine Phosphodiesterbindung miteinander verknüpft ist.

Bevorzugt ist das Polymer gemäß der Erfindung **dadurch gekennzeichnet, dass** die im Verfahren eingesetzten Desoxyribonukleinsäuren die folgende Sequenz umfassen:

·

5'-GGGTTACCACCTTCTATAGAAAACGTTCTTCGGGGCGTTCTTC-

ATCGGTAACCC-3' (SEQ ID Nr. 1)

· wobei die Desoxyribonukleinsäure eine Länge von 20 bis 400 Nukleotiden aufweist.

Die Synthese der Edukte mit den bevorzugten Sequenzen führt zu Molekülen, die überraschend gut zur Modulierung der Immunantwort eingesetzt werden können. Besonders bevorzugt ist es, wenn die Basenfolge in den einzelsträngigen Molekülbereichen der Sequenz
· 5'-TCATTGGAAACGTTCTTCGGGGCGTTCTT-3' (SEQ ID Nr. 2)
teilweise oder vollständig entspricht. Überraschenderweise führt das Vorhandensein dieser Sequenzen zu einer besonders guten Aktivität der concatemeren Polymere. Innerhalb der concatemeren Struktur der Moleküle sind die teilweise einzelsträngigen kovalent geschlossenen Ketten von Desoxyribonukleotiden dafür verantwortlich, dass die Moleküle in dem Zielorganismus, in den sie eingebracht werden, über einen längeren Zeitraum effektiv wirken.
In einer weiteren bevorzugten Ausführungsform der Erfindung ist vorgesehen, dass das Monomer die Basen-

folge $N^1N^2CGN^3N^4$ umfasst, wobei $N^1N^2$ ein Element aus der Gruppe GT, GG, GA, AT oder AA, $N^3N^4$ ein Element aus der Gruppe CT oder TT, sowie C Desoxycytosin, G Desoxyguanosin, A Desoxyadenosin und T Desoxythymidin ist.

In einer ganz besonders bevorzugten Ausführungsform ist vorgesehen, dass die Basenfolge $N^1N^2CGN^3N^4$ in dem einzelsträngigen Gruppen-Bereich W der geschlossenen Kette von Desoxyribonukleotiden positioniert ist. Insbesondere diese bevorzugten Moleküle zeigen eine sehr effektive und potente Wirkung bei der Modulierung des Immunsystems.

Selbstverständlich kann es vorgesehen sein, dass das erfindungsgemäße Molekül mit einem oder mehreren Substituenten durch kovalente Bindung verknüpft vorliegt. Bei diesen Substituenten kann es sich beispielsweise um Peptide, Proteine, Saccharide, Lipide, antigene Strukturen, DNA und/oder RNA handeln.

Die Erfindung betrifft neben den o. g. Struktur- und Funktionsmerkmalen des Produktes auch ein Verfahren zur Herstellung des Moleküls, das folgende Schritte umfasst:

- Bereitstellung eines Polyacrylamidgelelektrophorese-gereinigten, 5'-phosphorylierten DNA-Moleküls in Wasser,

- Lyophilisierung bei 50°C bis ein trockener Rückstand erhalten wird und anschließend Resuspension in einer Pufferlösung,

- Zugabe einer T4-DNA Ligase, wodurch ein Reaktionsgemisch gebildet wird, und

- Inkubation des Reaktionsgemisches bei 37°C für mindestens 30 Minuten,
  oder

- Bereitstellung des Desoxyribonukleinsäuremonomers nach einer Fällung und anschließende Trocknung des Präzipitats bei 50°C, oder Lyophylisierung des DNA-Molelüls bei 50°C in Gegenwart von Magnesiumchlorid

- Zugabe einer T4-DNA Ligase und

- Inkubation bei 37°C für mindestens 10 Minuten, bevorzugt für mindestens 30 Minuten.

Mit einer Fällung oder Lyophilisation in Gegenwart von Magnesiumchlorid werden die gleichen Ergebnisse in Bezug auf die Herstellung von Polymeren erzielt, insbesondere wenn die Desoxyribonukleinsäure mit einer Polyacrylamidgelelektrophorese gereinigt wurde, oder aber mit einer Kombination aus HPLC und FPLC.

Es war völlig überraschend, dass durch das Verfahren andere molekulare Strukturen als die Dimere, die im Stand der Technik (WO 2007/131495 oder WO 01/07055) beschrieben wurde, gewonnen werden konnten. Die Verfahren unterscheiden sich zwar nur in wenigen Schritten, umso überraschender war es aber, dass diese relativ geringfügigen Modifikationen der Herstellungsweise zu verschiedenen Molekülen führen. Die mit den Verfahren des Standes der Technik (WO 01/07055 oder WO 2007/131495) gewonnenen Strukturen weisen signifikante Unterschiede in ihren Eigenschaften auf. Die Moleküle unterscheiden sich deutlich in ihren immunmodulierenden Wirkungen, aber auch in anderen Charakteristika, wie z. B. den Nebenwirkungen. Neben den unterschiedlichen Verfahrensschritten führt auch die Verwendung von Edukten mit den bevorzugten Sequenzen zur Ausbildung von sehr spezifischen Reaktionsprodukten mit besonderen, überraschenden Eigenschaften. Die Verwendung der erfindungsgemäßen Sequenzen im Zusammenhang mit den genannten Verfahrensschritten führt zu besonders vorteilhaften Polymeren, die gegenüber denen im Stand der Technik bevorzugte Eigenschaften aufweisen.

Bevorzugt besteht das erfindungsgemäße Polymer aus 2+2n Monomeren (siehe Abb. 1), bevorzugt aus teilweise einzelsträngigen, kovalent geschlossenen Ketten aus Desoxyribonukleotidbausteinen, wobei die Monomeren einen Stammbereich und einen Loop-Bereich aufweisen, wobei der Stammbereich mindestens 2 Desoxyribonukleotide aufweist und der Loop-Bereich mind. 4 Desoxyribonukleotide, und im Loop-Bereich 1 bis 6 CG-Motive vorhanden sind und die Variable n ein Element aus der Menge der natürlichen Zahlen ist.

Die Erfindung betrifft auch ein Kombinationsmittel, welches mindestens ein erfindungsgemäßes Molekül und ein Chemotherapeutikum umfasst. Es war überraschend, dass die unerwartet starke Verbesserung der Immunantwort durch das erfindungsgemäße Molekül noch einmal deutlich verbessert werden kann, wenn das erfindungsgemäße Mittel mit bekannten Chemotherapeutika kombiniert wird und das Kombinationsmittel bevorzugt z. B. gegen Tumoren eingesetzt wird. Dem Fachmann war zwar bekannt, dass die Dimere gemäß der WO 01/07055 eine immunmodulierende Wirkung haben und es war weiterhin bekannt, dass Chemotherapeutika eine Wirkung auf Tumoren haben, es war aber überraschend, dass die aus den Monomeren gebildeten immunmodulatorischen Dimere zusammen mit den Chemotherapeutika einen überadditiven Effekt zeigten. Noch überraschender war, dass die aus den Monomeren gebildeten Polymere bzw. Concatemere in Kombination mit den Chemotherapeutika einen weit positiveren synergistischen Effekt zeigten als die Dimere. Die im erfindungsgemäßen Kombinationsmittel vereinigten Elemente wirken auf das einheitliche Ziel der Bekämpfung von Pathogenen, insbesondere von Tumoren hin. Bei dem erfindungsgemäßen Kombinationsmittel entwickelt jedes Element nicht nur seine bekannten Einzelwirkungen, sondern durch die Wechselwirkung der einzelnen Elemente wird ein überraschend großer Gesamteffekt erzielt, der mit den Polymeren noch stärker ausgeprägt ist als mit den Dimeren. Das Kombinationsmittel im Sinne der Erfindung kann auch als Kit vorliegen, in welchem das erfindungsgemäße Molekül und das Chemotherapeutikum gemäß des Standes der Technik getrennt vorliegen. So können in bevorzugten Ausführungsformen die mindestens zwei Bestandteile des Kits zeitgleich oder zeitversetzt appliziert werden. Beispielsweise kann die Gabe des erfindungsgemäßen Kombinationsmittels das Immunsystem so aktivieren, dass eine nachfolgende Applikation eines Chemotherapeutikums seine Wirkung besonders effektiv entfalten kann. Selbstverständlich ist es aber auch möglich, dass zunächst das Chemotherapeutikum appliziert und anschließend zeitlich versetzt davon das erfindungsgemäße Molekül in den humanen oder tierischen Organismus gegeben wird. Bei bestimmten Tumoren ist die zeitgleiche Gabe von erfindungsgemäßem Molekül und Chemotherapeutikum bevorzugt.

In einer bevorzugten Ausführungsform der Erfindung ist das Chemotherapeutikum ausgewählt aus der Gruppe umfassend Antikörper, Alkylantien, Platinanaloga, Interkalantien, Antibiotika, Mitosehemmer, Taxane, Topoisomerasehemmer, Antimetabolite und/oder L-Asparaginase, Hydroxycarbamid, Mitotane und/oder Amanitine.

In einer bevorzugten Ausführungsform der Erfindung sind die Alkylantien ausgewählt aus der Gruppe umfassend

- Stickstoff-Lost-Derivate, insbesondere

- Cyclophosphamid ,

- Ifosfamid,

- Trofosfamid,

- Melphalan und/oder

- Chlorambucil

- Akylsulfonate, insbesondere

- Busulfan und/oder

- Treosulfan

- Nitrosoharnstoffe, insbesondere

- Carmustin,

- Lomustin,

- Nimustin,

- Estramustin und/oder

- Streptozotocin

- Procarbazin und Dacarbazin,

- Temozolomid und/oder

- Thiotepa.

Die Alkylantien wirken besonders gut auf Tumoren, wodurch sie deren Wachstum inhibieren.

In einer bevorzugten Ausführungsform der Erfindung sind die Platinanaloga ausgewählt sind aus der Gruppe umfassend:

- Cisplatin,

- Carboplatin und/oder

- Oxaliplatin.

In einer weiteren bevorzugten Ausführungsform der Erfindung ist vorgesehen, dass die interkalierenden Substanzen ausgewählt sind aus der Gruppe umfassend:

- Anthracycline, insbesondere

- Doxorubicin (Adriamycin),

- Daunorubicin,

- Epirubicin und/oder

- Idarubicin,

- Mitoxantron,

- Amsacrin und/oder

- Doxifluridin

In einer weiteren bevorzugten Ausführungsform der Erfindung ist vorgesehen, dass die Antibiotika ausgewählt sind aus der Gruppe umfassend:

- Bleomycin,

- Actinomycin D (Dactinomycin) und/oder

- Mitomycin

Weiterhin kann es in einer anderen bevorzugten Ausführungsform der Erfindung vorteilhaft sein, dass die Mitosehemmer ausgewählt sind aus der Gruppe umfassend:

- Alkaloide der Vinca rosea, insbesondere

- Vinorelbin,

- Vincristin (Oncovin),

- Vinblastin und/oder

- Vindesin

In einer weiter besonders bevorzugten Ausführungsform der Erfindung sind die Taxane ausgewählt aus der Gruppe umfassend:

- Paclitaxel und/oder

- Docetaxel.

Weiterhin kann es bevorzugt sein, dass die Topoisomerasehemmer ausgewählt sind aus der Gruppe umfassend:

- Topoisomerase-I-Inhibitoren, insbesondere

- Camptothecin,

- Topotecan und/oder

- Irinotecan und/oder

- Topoisomerase-II-Inhibitoren, insbesondere

- Etoposid

- Teniposid.

Weiterhin ist es bevorzugt, dass in einer besonderen Ausführungsform der Erfindung die Antimetabolite ausgewählt sind aus der Gruppe umfassend:

- Folsäureantagonist, insbesondere

  - Methotrexat,

- Pyrimidianaloga, insbesondere

  - 5-Fluorouracil,

  - Capecitabin,

  - Cytosinarabinosid (Cytarabin) und/oder

  - Gemcitabin,

- Purinanaloga, insbesondere

  - 6-Thioguanin,

  - Pentostatin,

  - Azathioprin,

  - 6-Mercaptopurin,

  - Fludarabin und/oder

  - Cladribin.

Die Erfindung betrifft auch einen Kit, der das erfindungsgemäße Molekül und das Chemotherapeutikum umfasst, gegebenenfalls mit einer Information zum Kombinieren der Inhalte des Kits. Die Erfindung betrifft aber auch - wie bereits ausgeführt - ein pharmazeutisches Mittel, welches das erfindungsgemäße Molekül oder das Kombinationsmittel gegebenenfalls mit einem pharmazeutisch verträglichen Träger umfasst.

Die Erfindung betrifft weiterhin die Verwendung des Moleküls, des Kombinationsmittel oder des pharmazeutischen Mittels zur Herstellung eines Mittels zur Modulation eines menschlichen oder tierischen Immunsystems oder zur Modulation der Aktivität dieses Immunsystems. Unter Modulation des menschlichen oder tierischen Immunsystems wird jeder Einfluss auf das Immunsystem verstanden, der dazu führt, dass das Immunsystem auf Tumoren bzw. auf Krebs insbesondere inhibierend wirkt. Die Modulation der Aktivität des Immunsystems kann hierzu synonym verstanden werden oder sie beschreibt die dem Fachmann bekannten Aktivitäten des Immunsystems, die gegen Tumoren gerichtet sind und die durch die erfindungsgemäßen Mittel in Ihre Wirkung überraschend gesteigert werden. Die Modulation ist daher insbesondere eine Stimulierung oder Steigerung von Wirkungen des Immunsystems bzw. des Immunsystems selbst im Sinne einer tumorsuppressiven, -remittierenden oder prophylaktischen Wirkung. So können die erfindungsgemäßen Mittel in einer bevorzugten Ausführungsform dazu verwendet werden, die T-Zell vermittelte Immunantwort, aber auch die T-Zell unabhängige Immunantwort zu verändern. Dieser Vorgang kann in einer bevorzugten Ausführungsform der Erfindung eine Proliferation von B-Zellen umfassen oder eine B-Zell-Aktivierung.

In einer ganz besonders bevorzugten Ausführungsform kommt es bei der Modulation der Aktivität des Immunsystems zu einer Verbesserung dergestalt, dass die Cytokin-Sekretion verschiedener immunologisch relevanter Zellpopulationen verändert bzw. revertiert wird. Es kann besonders bevorzugt sein, wenn das erfindungsgemäße Molekül bzw. das Kombinationsmittel gemäß der Erfindung als Adjuvans in der

therapeutischen oder prophylaktischen Vakzinierung eingesetzt werden. Besonders wirksam können die erfindungsgemäßen Mittel zur Behandlung einer Zellwachstumsstörung eingesetzt werden, wobei in einer bevorzugten Ausführungsform die Zellwachstumsstörung eine Tumorerkrankung ist. Bevorzugt handelt es sich bei der Tumorerkrankung um eine Krankheit, die ausgewählt ist aus der Gruppe umfassend

Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome,

Tumoren der Lunge umfassend nicht-kleinzellige Bronchialkarzinome, kleinzellige Bronchialkarzinome, Tumoren des Mediastinums,

Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome,

Urogenitaltumoren umfassend Tumoren der Nieren, der Harnleiter, der Blase, der Prostata, der Harnröhre, des Penis und der Hoden,

gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii),

Tumoren der Bauchhöhle,

Mammakarzinome,

Tumoren endokriner Organe umfassend Tumoren der Schilddrüse, der Nebenschilddrüse, der Nebennierenrinde, endokrine Pankreastumoren,

Karzinoidtumoren und Karzinoidsyndrom,

multiple endokrine Neoplasien,

Knochen- und Weichteilsarkome, Mesotheliome,

Hauttumoren, Melanome umfassend kutane und intraokulare Melanome,

Tumoren des zentralen Nervensystems,

Tumoren im Kindesalter umfassend Retinoblastom, Wilms Tumor, Neurofibromatose, Neuroblastom, Ewing-Sarkom Tumorfamilie, Rhabdomyosarkom,

Lymphome umfassend Non-Hodgkin-Lymphome, kutane T-Zell-Lymphome, primäre Lymphome des zentralen Nervensystems, Morbus Hodgkin,

Leukämien umfassend akute Leukämien, chronische myeloische und lymphatische Leukämien, Plasmazell-Neoplasmen, myelodysplastische Syndrome, paraneoplastische Syndrome,

Metastasen ohne bekannten Primärtumor (CUP-Syndrom),

metastasierte Tumoren umfassend Gehirnmetastasen, Lungenmetastasen, Lebermetastasen, Knochenmetastasen, pleurale und perikardiale Metastasen und maligne Aszites,

peritoneale Karzinomastose,

Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren,

Transplantations-bezogene Malignitäten.

Im Folgenden soll die Erfindung an Hand von Beispielen näher erläutert werden, ohne auf diese Beispiele beschränkt zu sein.
Beispiel für die Herstellung der immunmodulatorischen Nukleinsäuresemoleküle:

a) Herstellung des hier nicht beanspruchten dimeren Moleküls:
5'-Phosphoryliertes Desoxyribonukleotidmolekül der Sequenz 5'-CCTAGGGGTT-ACCACCTTCATTGGAAAACGTTCTTCGGGGCGTTCTTAGGTGGTAACC-3' (SEQ ID Nr. 3) wurden 5 min auf 90°C erhitzt und anschließend auf Eis gekühlt, um die Ausbildung der Haarnadelstruktur zu ermöglichen. Selbstkomplementäre Überhänge wurden bei einer Endkonzentration von 1 mg/ml DNA in Gegenwart von T4-DNA Ligase (0,1 U/μg ODN) 24 h bei 37°C ligiert. Auftrennung des gereinigten Ligationsproduktes in 3 % Agarosegel, siehe Abb. 2, Spur 2.

b) Herstellung einer Tetramers, beispielhaft für die beanspruchten Polymere:

Der Grad der Polymerisierung kann durch die Konzentration des eingesetzten 5'-phosphorylierten Nukleinsäure nur begrenzt beeinflußt werden. Für die gezielte Herstellung eines dimeren Concatemers, wie es in Abb. 2 Spur 3 dargestellt ist, wurde deshalb das Herstellungsverfahren wie folgt modifiziert:

- 5'-phosphorylierte Nukleinsäuren der Sequenz 5'-CCCTAGGGGTTACC-ACCTTCATTGGAAAACGTTCTTCGGGGCGTTCTTTCCCCAATGG-TGGA-3' (SEQ ID Nr. 4) und 5'-CCCTTCCACCATTGGGGATCATTGG-AAAACGTTCTTCGGGGCGTTCTTAGGTGGTAACCCCT-3' (SEQ ID Nr. 5) wurden für 5 min in äquimolaren Konzentra-tionen (50 μM) bei 95°C denaturiert, anschließend langsam über 50 min auf 25°C abgekühlt

- hierzu wurden in einfachem molaren Überschuß das 5'-phosphorylierte Nukleinsäuren der Sequenz 5'-AGGGGTTACCACCTTCATTGGAAAA-CGTTCTTCGGGGCGTTCTTAGGTGGTAAC-3' (SEQ ID Nr. 6) gegeben und ligiert;

- die weiteren Schritte erfolgten nach dem oben beschriebenen allgemeinen Herstellungsverfahren.

Auftrennung des gereinigten Ligationsproduktes in 3 % Agarosegel, siehe Abb. 2, Spur 3.

c) Herstellung von höhermolekularen Polymeren:

Die Nukleinsäure mit der Sequenz 5'-pCTAGGGGTTACCACCTACAAAA-AAA-AACGAAATTCGGGGCGAAGGGAGGTGGTAACCC-3' (SEQ ID Nr. 7) mit der Konzentration 1 mg/ml wurde mit 0,3 M Natrium-Acetat (pH 5,25), 10 mM MgCl$_2$ und einem dreifachen Volumen Ethanol gefällt. Nach Zentrifugation (4°C, 13000 rpm) wurde das ODN bei 50°C für 10 min getrocknet. Das Pellet wurde direkt in den Ligationsansatz (0,5 U/μg ODN) eingesetzt und 60 min bei 37°C inkubiert.
Auftrennung der gereinigten Ligationsprodukte in 3 % Agarosegel, siehe Abb. 2, Spur 4.

Beschreibung der Abb.2:

Zur Bestimmung der Molekulargewichte wurden die hergestellten Moleküle auf einem 3 % AgaroseGel aufgetrennt. Auf der linken Spur 1 sind die Molekulargewichte von doppelsträngiger DNA aufgeführt und den DNA-Banden jeweiliger Größe, erkennbar an den unterschiedlichen Migrationsstrecken, zugeordnet. In den Spuren 2-4 wurden die Produkte der verschiedenen Polymerisationsreaktionen aufgetragen. Es zeigt sich eine Einzelbande, die dem Dimer (Spur 2) bzw. einem Tetramer (Spur 3) entspricht bzw. eine Leiter, die alle Polymerenformen umfasst (Spur 4).

Funktionsnachweis der erfindungsgemäßen Moleküle:

Es wurden verschiedene Zellkulturexperimente, die die immunmodulierenden Eigenschaften der erfindungsgemäßen Moleküle darstellen, durchgeführt. Die Fähigkeit zur Stimulation des TLR9 wurde mit Hilfe der murinen Makrophagen Zelllinie RAW 264 untersucht, in der die Expression des grünfluoreszierenden Proteins EGFP unter der Kontrolle des positiv durch TLR9 regulierten NF|B-Promoters steht. Die Zellen wurden dafür als 125000 Zellen/cm$^2$ ausgesät und nach 16 h mit den dimeren (nach Verfahren a hergestellt) und den polymeren (nach Verfahren c hergestellt) erfindungsgemäßen Molekülen versetzt. Nach 7 h Inkubation (37°C, 5 % $CO_2$ wurden die Zellen geerntet und die EGFP-Expression im Durchflußcytometer bestimmt. Aus den Ergebnissen wurde eine Konzentrations-Wirkungs-Kurve erstellt, die in Abb. 3 dargestellt ist; als Molekulargewicht wurde für beide Molekülgruppen das der Dimere zugrunde gelegt, um die direkte Vergleichbarkeit zu ermöglichen .

Es zeigt sich eine um den Faktor 10 erhöhte Potenz der erfindungsgemäßen polymeren Molekülen (obere Kurve mit geschlossenen Symbolen) im Vergleich zu den niedermolekularen Dimeren (untere gestrichelte Kurve mit offenen Symbolen). Die erfindungsgemäßen höhermolekularen Polymere haben damit eine deutlich größere Wirksamkeit bei gleicher eingesetzter Menge als entsprechende Mengen der dimeren oder monomeren Moleküle. Diese höhere Potenz bei der TLR9-Stimulation kann darauf zurückgeführt werden, daß durch die polymeren Moleküle eine lokal höhere Konzentration erreicht wird, die besonders *in vivo* nicht durch eine Erhöhung der Dosis erreicht werden kann, aus Gründen bspw. der applizierbaren Menge. Gleichzeitig zeigen die höhermolekularen Concatemere auch eine gesteigerte Effizienz, die völlig überraschend bei den Experimenten beobachtet wurde und nach aktuellem Stand der Wissenschaft nicht erklärbar ist.

Stimulation von PBMCs zur Cytokinproduktion

Zur Durchführung der Stimulationsversuche wurden periphere mononukleäre Blutzellen (PBMC) aus menschlichem Vollblut oder sogenannten "Buffy Coat" gewonnen. Die isolierten Zellen (PBMC) wurden in Multi-well-plates ausgesät. Im ersten Ansatz befanden sich als negative Kontrolle unstimulierte Zellen, der zweite Ansatz wurde als Vergleich zum Stand der Technik mit den Dimeren stimuliert, der dritte mit einem tetrameren Polymer; es wurden jeweils gleichen Massen-Mengen an Dimer bzw. Polymer in gleichen Volumina eingesetzt. Die Ausschüttung der Cytokine Interferon-y, Interferon-$\alpha$ und Interleukin-6 wurde 2 Tage später mittels ELISA aus dem Zellkulturüberstand bestimmt, siehe Abb. 4.

Entsprechend Abb. 4 wird durch die Stimulation von PBMCs mit den erfindungsgemäßen polymeren Molekülen eine Verdopplung der Interferon-Sekretion im Vergleich zur Stimulation durch dimere Moleküle erreicht.

Die Abb. zeigt weiterhin, daß die IL-6 Sekretion infolge der Stimulation mit polymeren Molekülen signifikant stärker erfolgt als nach Stimulation mit Dimeren.

Erfindungsgemäße Moleküle können z.B. aus Monomeren mit folgender Basensequenz hergestellt werden:

a)

5'-CTAGGGGTTACCACCTTCTATAGAAAACGTTCTTCGGGGCGTT-

CTTCATCGGTAACCC-3' (SEQ ID Nr. 8) oder

b)

5'-AGCTGGGTTACCACCTTCATTGGAAAACGTTCTTCGGGGCGTT-

CTTAGGTGGTAACCC-3' (SEQ ID Nr. 9) oder

c) jeder 5'-terminal phosphorylierten Nukleinsäure oder Gemischen aus Nukleinsäuren, deren Sequenzen die in Abb. 1 dargestellten Konformationen einnehmen können, den dargestellten Komplementaritätskriterien genügen und durch geeignete mindestens 4 Nukleotide lange einzelsträngige Überhänge ("sticky ends") miteinander hybridisieren können.

Die Edukt-Desoxyribonukleinsäuren werden vor der Ligation nicht erhitzt und liegen mit dem Reinigungsgrad einer Polyacrylamidgelelektrophorese vor. Die Edukte können auch mittels HPLC gefolgt von einem weiteren Chromatographieschritt (FPLC) gereinigt sein. Durch die Kombination von HPLC und FPLC wird ein äquivalenter Reinigungsgrad zu einer Polyacrylamidgelelektrophorese erreicht. Anschließend werden die DNA-Edukte so lange bei 50°C lyophilisiert, bis ein trockener Rückstand erhalten wird. Anschließend wird eine Resuspension in Pufferlösung durchgeführt und es erfolgt die Zugabe einer T4-DNA Ligase mit anschließender Inkubation bei 37°C für 40 Minuten. Es zeigt sich überraschend, dass die gewonnenen Concatemere zu einer verbesserten Immunmodulation in Mäusen führen. Überraschenderweise führt die Kombination der erfindungsgemäßen Concatemere mit Chemotherapeutika zu einer verbesserten Wirkung. Diese verbesserte Wirkung ist überraschend größer als die der Einzelverbindung und geht über einen additiven Effekt hinaus. Als Chemotherapeutika können Antikörper, Alkylantien, Platinanaloga, Interkalantien, Antibiotika, Mitosehemmer, Taxane, Topoisomerasehemmer, Antimetabolite, L-Asparaginase, Hydroxycarbamid, Mitotane oder Amanitine verwendet werden.

**Patentansprüche**

1.  Concatemeres Molekül zur Modulation der Aktivität des menschlichen oder tierischen Immunsystems, wobei das concatemere Molekül ein Molekül ist, bei dem mindestens vier Monomereinheiten aus Desoxyribonukleinsäuren kovalent miteinander verbunden sind und der allgemeinen Formel genügen:

$$B\text{-}M\text{-}\{[J_i\text{-}U_i\text{-}A_i]_{0..n}\}_n\text{-}E\text{-}K\text{-}T\text{-}\{[R_{n-i+1}\text{-}Y_{n-i+1}\text{-}I_{n-i+1}]_{0..n}\}_n\text{-}G$$

$\forall\, n \in IN_0$ , wobei

- A, B, E, G, I, J, K, M, R, T, U, Y Desoxyribonukleotid-Moleküle sind und
- "-" eine Phosphodiesterbindung darstellt, über die die Nukleinsäuren kovalent miteinander verbunden sind und
- die Sequenz des i-ten Bausteins eines Nukleinsäuremoleküls von der (i+1)-ten des gleichen Moleküls verschieden sein kann aber nicht muß, und
- mindestens eine der Nukleinsäuren ein Motiv mit der Desoxyribonukleotidabfolge CG enthält und
- B, $U_i$, K und $Y_{n-i+1}$ überwiegend einzelsträngig vorliegen und
- B, $U_i$, K und $Y_{n-i+1}$ aus jeweils mindestens 4 Desoxyribonukleotiden aufgebaut sind und
- sich die Sequenzen von $J_i$ zu $I_{n-i+1}$, $A_i$ zu $R_{n-i+1}$, M zu G bzw. E zu T revers kompementär zueinander verhalten, miteinander hybridisieren und eine doppelsträngige Substruktur ausbilden können und
- G mit B kovalent über eine Phosphodiesterbindung miteinander verknüpft sind.

2. Molekül nach dem vorhergehenden Anspruch,
**dadurch gekennzeichnet, dass**
die Desoxyribonukleinsäuren folgende Sequenzen umfassen:

.

5'-GGGTTACCACCTTCATTGGAAAACGTTCTTCGGGGCGTTCTTA-GGTGGTAACCC-3' (SEQ ID Nr. 1) oder

.

5'-CCCTAGGGGTTACCACCTTCATTGGAAAACGTTCTTCGGGGC-GTTCTTTCCCCAATGGTGGA-3' (SEQ ID Nr. 4) oder

.

5'-CCCTTCCACCATTGGGGATCATTGGAAAACGTTCTTCGGGGC-GTTCTTAGGTGGTAACCCCT-3' (SEQ ID Nr. 5) oder

.

5'-AGGGGTTACCACCTTCATTGGAAAACGTTCTTCGGGGCGTTCT-TAGGTGGTAAC-3' (SEQ ID Nr. 6),

wobei die Desoxyribonukleinsäure eine Länge von 20 bis 400 Nukleotiden aufweist.

3. Kombinationsmittel umfassend ein Molekül nach einem der Ansprüche 1 oder 2 und ein Chemotherapeutikum, ausgewählt aus der Gruppe umfassend Antikörper, Alkylantien, Platinanaloga, Interkalantien, Antibiotika, Mitosehemmer, Taxane, Topoisomerasehemmer, Antimetabolite und/oder L-Asparaginase, Hydroxycarbamid, Mitotane und/oder Amanitine.

4. Kombinationsmittel nach dem vorhergehenden Anspruch,
**dadurch gekennzeichnet, dass**
die Alkylantien ausgewählt sind aus der Gruppe umfassend:

- Stickstoff-Lost-Derivate, insbesondere

- Cyclophosphamid,
- Ifosfamid,
- Trofosfamid,
- Melphalan und/oder
- Chlorambucil

- Akylsulfonate, insbesondere

- Busulfan und/oder
- Treosulfan

- Nitrosoharnstoffe, insbesondere

- Carmustin,
- Lomustin,
- Nimustin,
- Estramustin und/oder
- Streptozotocin

- Procarbazin und Dacarbazin,
- Temozolomid und/oder
- Thiotepa.

5. Kombinationsmittel nach einem der vorhergehenden Ansprüche, **dadurch gekennzeichnet, dass** Platinanaloga ausgewählt sind aus der Gruppe umfassend:

- Cisplatin,
- Carboplatin und/oder
- Oxaliplatin.

6. Kombinationsmittel nach einem der vorhergehenden Ansprüche, **dadurch gekennzeichnet, dass** die Interkalatoren ausgewählt sind aus der Gruppe umfassend:

- Anthracycline, insbesondere

- Doxorubicin (Adriamycin),
- Daunorubicin,
- Epirubicin und/oder
- Idarubicin,

- Mitoxantron,
- Amsacrin und/oder
- Doxifluridin.

7. Kombinationsmittel nach einem der vorhergehenden Ansprüche, **dadurch gekennzeichnet, dass** die Antibiotika ausgewählt sind aus der Gruppe umfassend:

- Bleomycin,

- Actinomycin D (Dactinomycin) und/oder
- Mitomycin.

**8.** Kombinationsmittel nach einem der vorhergehenden Ansprüche, **dadurch gekennzeichnet, dass** die Mitosehemmer ausgewählt sind aus der Gruppe umfassend:

- Alkaloide der Vinca rosea, insbesondere

- Vinorelbin,
- Vincristin (Oncovin),
- Vinblastin und/oder
- Vindesin.

**9.** Kombinationsmittel nach einem der vorhergehenden Ansprüche, **dadurch gekennzeichnet, dass** die Taxane ausgewählt sind aus der Gruppe umfassend:

- Paclitaxel und/oder
- Docetaxel.

**10.** Kombinationsmittel nach einem der vorhergehenden Ansprüche, **dadurch gekennzeichnet, dass** die Topoisomerasehemmer ausgewählt sind aus der Gruppe umfassend:

- Topoisomerase-I-Inhibitoren, insbesondere

- Camptothecin,
- Topotecan und/oder
- Irinotecan und/oder

- Topoisomerase-II-Inhibitoren, insbesondere

- Etoposid
- Teniposid.

**11.** Kombinationsmittel nach einem der vorhergehenden Ansprüche, **dadurch gekennzeichnet, dass** die Antimetabolite ausgewählt sind aus der Gruppe umfassend:

- Folsäureantagonist, insbesondere

- Methotrexat,

- Pyrimidianaloga, insbesondere

- 5-Fluorouracil,
- Capecitabin,
- Cytosinarabinosid (Cytarabin) und/oder
- Gemcitabin,

- Purinanaloga, insbesondere

- 6-Thioguanin,
- Pentostatin,
- Azathioprin,
- 6-Mercaptopurin,
- Fludarabin und/oder
- Cladribin.

**12.** Kit umfassend das Molekül nach einem der Ansprüche 1 oder 2 und/oder das Kombinationsmittel nach einem der Ansprüche 3 bis 11 und ggf. eine Information zum Kombinieren der Inhalte des Kit.

**13.** Molekül nach einem der Ansprüche 1 oder 2, Kombinationsmittel nach einem der Ansprüche 3 bis 11 zur Verwendung als Arzneimittel.

**14.** Pharmazeutisches Mittel umfassend ein Molekül nach einem der Ansprüche 1 oder 2 und/oder ein Kombinationsmittel nach einem der Ansprüche 3 bis 11 gegebenenfalls mit einem pharmazeutisch verträglichen Träger.

**15.** Pharmazeutisches Mittel nach dem vorhergehenden Anspruch,
**dadurch gekennzeichnet, dass**
der Träger ausgewählt ist aus der Gruppe umfassend Antikörper, Alkylantien, Platinanaloga, Interkalantien, Antibiotika, Mitosehemmer, Taxane, Topoisomerasehemmer, Antimetabolite und/oder L-Asparaginase, Hydroxycarbamid, Mitotane, und/oder Amanitine.

**16.** Verwendung eines Moleküls gemäß der Ansprüche 1 oder 2, eines Kombinationsmittel gemäß eines der Ansprüche 3 bis 11 und/oder eines pharmazeutischen Mittels gemäß Anspruch 14 oder 15 zur Herstellung eines Mittels zur Modulation eines menschlichen oder tierischen Immunsystems oder zur Modulation des Immunsystems.

**17.** Verwendung nach dem vorhergehenden Anspruch,
**dadurch gekennzeichnet, dass**
die Modulation eine Verbesserung der Aktivität des Immunsystems ist, wobei die Aktivität einzelner Zellen oder Zell-Subpopulationen des Immunsystems stimuliert oder beschleunigt oder inhibiert oder attenuiert sein können.

**18.** Verwendung nach dem vorhergehenden Anspruch,
**dadurch gekennzeichnet, dass**
die Modulation eine T-Helferzell-vermittelte oder-unabhängige Immunantwort umfasst.

**19.** Verwendung nach dem vorhergehenden Anspruch,
**dadurch gekennzeichnet, dass**
die Immunantwort eine Proliferation von B-Zellen umfasst und/oder eine B-Zell-Aktivierung.

**20.** Verwendung nach einem der vorhergehenden Ansprüche,
**dadurch gekennzeichnet, dass**
die Modulation des Immunsystems eine Sekretion von Cytokinen umfasst.

**21.** Verwendung nach einem der vorhergehenden Ansprüche,
**dadurch gekennzeichnet, dass**
das Molekül gemäß eines der Ansprüche 1 oder 2 und/oder das Kombinationsmittel gemäß eines der Ansprüche 3 bis 11 als Adjuvans in der therapeutischen oder prophylaktischen Vakzinierung eingesetzt wird.

**22.** Verwendung eines Moleküls gemäß eines der Ansprüche 1 oder 2, eines Kombinationsmittels gemäß eines der Ansprüche 3 bis 11 und/oder eines pharmazeutischen Mittels gemäß Anspruch 14 oder 15 zur Herstellung eines Mittels zur Behandlung einer Zellwachstumsstörung.

**23.** Verwendung nach dem vorhergehenden Anspruch,
**dadurch gekennzeichnet, dass**
die Zellwachstumsstörung eine Tumorerkrankung ist.

**24.** Verwendung nach dem vorhergehenden Anspruch,
**dadurch gekennzeichnet, dass**
die Tumorerkrankung ausgewählt ist aus der Gruppe umfassend Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehirnmetastasen, Lungen-metastasen, Lebermetastasen, Knochenmetastasen, pleurale und perikardiale Metastasen und maligne Aszites.

Abb. 1

für i: 0..n    n ∈ IN₀    ·············· kovalente Bindung

Abb. 2:

Abb. 3:

Abb. 4:

| Europäisches Patentamt European Patent Office Office européen des brevets | **EUROPÄISCHER RECHERCHENBERICHT** | **Nummer der Anmeldung** EP 09 07 5220 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2007/131495 A2 (MOLOGEN AG [DE]; SCHROFF MATTHIAS [DE]; WITTIG BURGHARDT [DE]; SCHMIDT) 22. November 2007 (2007-11-22) * das ganze Dokument * ----- | 1-17,20 | INV. C12N15/117 A61K31/713 |
| A | WO 2006/015560 A1 (MOLOGEN AG [DE]; WITTIG BURGHARDT [DE]; SCHMIDT MANUEL [DE]; BOHLEN HE) 16. Februar 2006 (2006-02-16) * das ganze Dokument * ----- | 1-17,20 | |
| A,D | WO 01/07055 A1 (MOLOGEN FORSCHUNGS ENTWICKLUNG [DE]; JUNGHANS CLAAS [DE]; WITTIG BURGH) 1. Februar 2001 (2001-02-01) * das ganze Dokument * ----- | 1-17,20 | |
| X | SEYHAN ATTILA A ET AL: "RNA interference from multimeric shRNAs generated by rolling circle transcription" OLIGONUCLEOTIDES, Bd. 16, Nr. 4, 2006, Seiten 353-363, XP002567312 ISSN: 1545-4576 * Abbildung 3 * ----- | 1,12-14, 16 | RECHERCHIERTE SACHGEBIETE (IPC) C12N A61K |
| E | WO 2009/059805 A1 (MOLOGEN AG [DE]; SCHROFF MATTHIAS [DE]; WITTIG BURGHARDT [DE]; SCHMIDT) 14. Mai 2009 (2009-05-14) * das ganze Dokument * ----- | 1-17,20 | |

Der vorliegende Recherchenbericht wurde für alle Patentansprüche erstellt

| Recherchenort | Abschlußdatum der Recherche | Prüfer |
|---|---|---|
| Den Haag | 8. Februar 2010 | Andres, Serge |

EPO FORM 1503 03.82 (P04C03)

---

## GEBÜHRENPFLICHTIGE PATENTANSPRÜCHE

Die vorliegende europäische Patentanmeldung enthielt bei ihrer Einreichung Patentansprüche, für die eine Zahlung fällig war.

[X] Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

16-17, 20

[ ] Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

---

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

[ ] Alle weiteren Recherchengebühren wurden innerhalb der gesetzten Frist entrichtet. Der vorliegende europäische Recherchenbericht wurde für alle Patentansprüche erstellt.

[ ] Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

[ ] Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

[ ] Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

[ ] Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

**ANHANG ZUM EUROPÄISCHEN RECHERCHENBERICHT
ÜBER DIE EUROPÄISCHE PATENTANMELDUNG NR.**

EP 09 07 5220

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-02-2010

| Im Recherchenbericht angeführtes Patentdokument | Datum der Veröffentlichung | Mitglied(er) der Patentfamilie | Datum der Veröffentlichung |
|---|---|---|---|
| WO 2007131495 A2 | 22-11-2007 | AU 2007250331 A1<br>CA 2651568 A1<br>CN 101490257 A<br>EA 200802310 A1<br>EP 2027266 A2<br>JP 2009536519 T<br>KR 20090012265 A | 22-11-2007<br>22-11-2007<br>22-07-2009<br>30-06-2009<br>25-02-2009<br>15-10-2009<br>02-02-2009 |
| WO 2006015560 A1 | 16-02-2006 | EP 1776124 A1<br>WO 2006015872 A1<br>US 2009053250 A1 | 25-04-2007<br>16-02-2006<br>26-02-2009 |
| WO 0107055 A1 | 01-02-2001 | AT 268600 T<br>AU 3418000 A<br>DE 10082079 D2<br>DE 19935756 A1<br>DK 1196178 T3<br>EP 1196178 A1<br>ES 2222894 T3<br>PT 1196178 E<br>US 2003125279 A1 | 15-06-2004<br>13-02-2001<br>14-08-2002<br>08-02-2001<br>27-09-2004<br>17-04-2002<br>16-02-2005<br>29-10-2004<br>03-07-2003 |
| WO 2009059805 A1 | 14-05-2009 | EP 2058397 A1 | 13-05-2009 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

### In der Beschreibung aufgeführte Patentdokumente

- WO 0107055 A
- EP 1196178 A
- WO 2007131495 A

### In der Beschreibung aufgeführte Nicht-Patentliteratur

- **Krieg.** *Nat. Med,* 2003, vol. 9, 831-835

SEQUENCE LISTING

<110> MOLOGEN AG

<120> Concatemere zur Immunmodulation

<130> XI 411/09

<160> 9

<170> PatentIn version 3.5

<210> 1
<211> 54
<212> DNA
<213> Artificial Sequence

<220>
<223> Synthetic Construct

<400> 1
gggttaccac cttctataga aaacgttctt cggggcgttc ttcatcggta accc          54

<210> 2
<211> 29
<212> DNA
<213> Artificial Sequence

<220>
<223> Synthetic Construct

<400> 2
tcattggaaa cgttcttcgg ggcgttctt          29

<210> 3
<211> 58
<212> DNA
<213> Artificial Sequence

<220>
<223> Synthetic Construct

<400> 3
cctaggggtt accaccttca ttggaaaacg ttcttcgggg cgttcttagg tggtaacc          58

<210> 4
<211> 62
<212> DNA
<213> Artificial Sequence

<220>
<223> Synthetic Construct

<400> 4
ccctaggggt taccaccttc attggaaaac gttcttcggg gcgttctttc cccaatggtg          60

ga          62

<210> 5
<211> 62
<212> DNA
<213> Artificial Sequence

<220>

```
<223>   Synthetic Construct

<400>   5
cccttccacc attggggatc attggaaaac gttcttcggg gcgttcttag gtggtaaccc          60

ct                                                                          62


<210>   6
<211>   54
<212>   DNA
<213>   Artificial Sequence

<220>
<223>   Synthetic Construct,

<400>   6
aggggttacc accttcattg gaaaacgttc ttcggggcgt tcttaggtgg taac                54


<210>   7
<211>   58
<212>   DNA
<213>   Artificial Sequence

<220>
<223>   Synthetic Construct

<400>   7
ctaggggtta ccacctacaa aaaaaaacga aattcggggc gaagggaggt ggtaaccc            58


<210>   8
<211>   58
<212>   DNA
<213>   Artificial Sequence

<220>
<223>   Synthetic Construct

<400>   8
ctaggggtta ccaccttcta tagaaaacgt tcttcggggc gttcttcatc ggtaaccc            58


<210>   9
<211>   58
<212>   DNA
<213>   Artificial Sequence

<220>
<223>   Synthetic Construct

<400>   9
agctgggtta ccaccttcat tggaaaacgt tcttcggggc gttcttaggt ggtaaccc            58
```